(19) Europäisches Patentamt
European Patent Office
Office européen des brevets

(11) **EP 3 270 775 B1**

(12) **EUROPEAN PATENT SPECIFICATION**

(45) Date of publication and mention
of the grant of the patent:
**12.02.2020 Bulletin 2020/07**

(21) Application number: **16764295.8**

(22) Date of filing: **16.03.2016**

(51) Int Cl.:
*A61B 5/0444* (2006.01)    *A61B 5/0245* (2006.01)
*A61B 5/0402* (2006.01)

(86) International application number:
**PCT/IB2016/000406**

(87) International publication number:
**WO 2016/147051 (22.09.2016 Gazette 2016/38)**

(54) **SYSTEMS, APPARATUSES AND METHODS FOR SENSING FETAL ACTIVITY**

SYSTEME, VERFAHREN UND VORRICHTUNG ZUR MESSUNG DER FETALEN AKTIVITÄT

SYSTÈMES, APPAREILS ET PROCÉDÉS DE DÉTECTION DE L'ACTIVITÉ F TALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2015 US 201562133485 P**
**23.10.2015 US 201514921489**

(43) Date of publication of application:
**24.01.2018 Bulletin 2018/04**

(73) Proprietor: **Nuvo Group Ltd.**
**6520213 Tel Aviv (IL)**

(72) Inventors:
• **OZ, Oren**
**71720 Modi'in (IL)**
• **KAM, Amit**
**76450 Rehovot (IL)**
• **INTRATOR, Nathan**
**69120 Tel Aviv (IL)**

(74) Representative: **Trinks, Ole et al**
**Meissner Bolte Patentanwälte**
**Rechtsanwälte Partnerschaft mbB**
**Postfach 10 26 05**
**86016 Augsburg (DE)**

(56) References cited:
CN-A- 104 027 105    US-A1- 2007 260 155
US-A1- 2012 071 744    US-A1- 2014 005 988

• **VAISMAN SERGEY ET AL: "Passive fetal monitoring by adaptive wavelet denoising method", COMPUTERS IN BIOLOGY AND MEDICINE, vol. 42, no. 2, February 2012 (2012-02), pages 171-179, XP028885137, ISSN: 0010-4825, DOI: 10.1016/J.COMPBIOMED.2011.11.005**
• **NAJAFABADI F S ET AL: "Fetal heart rate monitoring based on independent component analysis", COMPUTERS IN BIOLOGY AND MEDICINE, NEW YORK, NY, US, vol. 36, no. 3, 1 March 2006 (2006-03-01), pages 241-252, XP028025040, ISSN: 0010-4825, DOI: 10.1016/J.COMPBIOMED.2004.11.004 [retrieved on 2006-03-01]**
• **VARADY P ED - INSTITUTE OF ELECTRICAL AND ELECTRONICS ENGINEERS: "Wavelet-based adaptive denoising of phonocardiographic records", 2001 CONFERENCE PROCEEDINGS OF THE 23RD ANNUAL INTERNATIONAL CONFERENCE OF THE IEEE ENGINEERING IN MEDICINE AND BIOLOGY SOCIETY (CAT. NO.01CH37272) VOL.3; [ANNUAL INTERNATIONAL CONFERENCE OF THE IEEE ENGINEERING IN MEDICINE AND BIOLOGY SOCIETY. EMBS], vol. 2, 25 October 2001 (2001-10-25), pages 1846-1849, XP010594795, ISBN: 978-0-7803-7211-5**

**Description**

FIELD OF THE INVENTION

The invention relates generally to systems and methods for monitoring the wellbeing of a fetus by the non-invasive detection and analysis of fetal cardiac activity data.

BACKGROUND

Monitoring fetal cardiac activity can be useful to determine the health of a fetus during pregnancy.

US 2014/0005988 A1 generally relates to physiological signal denoising. A denoised physiological signal is generated from an input signal including a desired physiological signal and noise. The input signal is decomposed from a first domain into sub-components in a second domain of a higher dimension than the first domain. Target sub-components of the input signal that are associated with the desired physiological signal are identified, based upon the spatial distribution of the sub-components. A denoised physiological signal is constructed in the first domain from at least one of the identified target sub-components.

US 2012/0071744 A1 relates to a sensor interface system for providing a connection between at least one sensor and a maternal-fetal monitor, wherein the interface system converts electrical muscle activity captured by the sensors into uterine activity data signals for use by the maternal-fetal monitor.

SUMMARY

In some embodiments, the instant invention is directed to a computer-implemented method which includes at least the steps of: receiving, by at least one computer processor executing specific programmable instructions configured for the method, a plurality of Phonocardiogram (PCG) signals data inputs from a plurality of acoustic sensors; digital signal filtering, by the at least one computer processor, utilizing a plurality of bandpass filters, the plurality of PCG signals data inputs to form a plurality of filtered PCG outputs, where the plurality of bandpass filters includes a L number of bandpass filters, where each bandpass filter outputs a K number of filtered PCG outputs; wavelet denoising, by the at least one computer processor, a first subset of filtered PCG outputs of the plurality of filtered PCG outputs to form a M number of denoised filtered PCG outputs, where M is equal to L multiply by K; transforming, by the at least one computer processor, utilizing an Independent-Component-Analysis (ICA), a second subset of filtered PCG outputs of the plurality of filtered PCG outputs to form the M number of filtered ICA transforms; transforming, by the at least one computer processor, utilizing the Independent-Component-Analysis (ICA), a first portion of the second subset of denoised filtered PCG outputs to form the M number of filtered denoised filtered ICA transforms; compiling, by the at least one computer processor, a S number of a plurality of detection heartbeat (DH) inputs, including: i) the M number of filtered PCG outputs, ii) the M number of denoised filtered PCG outputs, iii) the M number of filtered ICA transforms, and iv) the M number of denoised filtered ICA transforms; detecting, by the at least one computer processor, beat locations of beats in each of DH inputs; calculating, by the at least one computer processor, a confidence score that describes a probability that the beats in each DH input of the plurality of DH inputs represent actual heartbeats and not a noise; dividing, by the at least one computer processor, the plurality of DH inputs into at least two groups: i) a first group of DH inputs containing fetal heartbeats, ii) a second group of DH inputs containing maternal heartbeats selecting, by the at least one computer processor, from the first group of DH inputs, at least one particular fetal DH input that contains the fetal heartbeat based on a first confidence score of the at least one particular fetal DH input; and selecting, by the at least one computer processor, from the second group of DH inputs, at least one particular maternal DH input that contains the maternal heartbeat, based on a second confidence score of the at least one particular maternal DH input.

In some embodiments, where the wavelet denoising includes at least: deconstructing, by the at least one computer processor, each filtered PCG output to generate a plurality of transform coefficients, by irrelatively passing each filtered PCG output through a succession of low and high pass filters; identifying, by the at least one computer processor, a subset of heartbeat-carrying transform coefficients from the plurality of transform coefficients, reconstructing, by the at least one computer processor, the subset of heartbeat-carrying transform coefficients to form the M number of denoised filtered PCG outputs.

In some embodiments, K is equal to a number of the plurality of acoustic sensors, and L is equal to 6.

In some embodiments, each filter of the plurality of bandpass filters has a bandwidth of 10-100Hz and a frequency range of 5-110HZ.

In some embodiments, the detecting of the beat locations of the beats in each of DH inputs includes at least: calculating, by the at least one computer processor, a predetermined transform filter; iteratively repeating, by the at least one computer processor, based on a predetermined average window length of the predetermined transform filter: identifying, in each DH input, a subset of peaks having a predetermined shape and a predetermined size, and selecting a group of peaks from the subset of peaks, where the group of peaks has the smallest variance measure; calculating, by the at least one computer processor, initial locations of the beats.

In some embodiments, the calculating the confidence score includes at least: generating, by the at least one computer processor, a beat-by-beat heartbeat graph for each DH input based on the beat locations.

In some embodiments, the dividing the plurality of DH inputs into the first group of DH inputs containing fetal heartbeats and the second group of DH inputs containing maternal heartbeats includes at least: clustering, by the at least one computer processor, the beats of each DH input according to each beat value; and assigning, by the at least one computer processor, a particular DH input having a higher average beat rate into the first group of DH inputs containing fetal heartbeats.

In some embodiments, the present invention is directed to a specifically programmed computer system, including at least the following components: at least one specialized computer machine, including: a non-transient memory, electronically storing particular computer executable program code; and at least one computer processor which, when executing the particular program code, becomes a specifically programmed computing processor that is configured to at least perform the following operations: receiving a plurality of Phonocardiogram (PCG) signals data inputs from a plurality of acoustic sensors; digital signal filtering, utilizing a plurality of bandpass filters, the plurality of PCG signals data inputs to form a plurality of filtered PCG outputs, where the plurality of bandpass filters includes a L number of bandpass filters, where each bandpass filter outputs a K number of filtered PCG outputs; wavelet denoising a first subset of filtered PCG outputs of the plurality of filtered PCG outputs to form a M number of denoised filtered PCG outputs, where M is equal to L multiply by K; transforming, utilizing an Independent-Component-Analysis (ICA), a second subset of filtered PCG outputs of the plurality of filtered PCG outputs to form the M number of filtered ICA transforms; transforming, utilizing the Independent-Component-Analysis (ICA), a first portion of the second subset of denoised filtered PCG outputs to form the M number of denoised filtered ICA transforms; compiling a S number of a plurality of detection heartbeat (DH) inputs, including: i) the M number of filtered PCG outputs, ii) the M number of denoised filtered PCG outputs, iii) the M number of filtered ICA transforms, and iv) the M number of denoised filtered ICA transforms; detecting beat locations of beats in each of DH inputs; calculating a confidence score that describes a probability that the beats in each DH input of the plurality of DH inputs represent actual heartbeats and not a noise; dividing the plurality of DH inputs into at least two groups: i) a first group of DH inputs containing fetal heartbeats, ii) a second group of DH inputs containing maternal heartbeats; selecting, from the first group of DH inputs, at least one particular fetal DH input that contains the fetal heartbeat based on a first confidence score of the at least one particular fetal DH input; and selecting, from the second group of DH inputs, at least one particular maternal DH input that contains the maternal heartbeat, based on a second confidence score of the at least one particular maternal DH input.

## Brief Description of the Drawings

Figure 1 shows the positions of the exemplary acoustic sensors on the abdomen of a pregnant woman according to some embodiments of the present invention.

Figure 2 shows a flow chart of an algorithm used to detect and analyze cardiac activity data according to some embodiments of the present invention.

Figures 3A and 3B show flow charts of a signal denoising algorithm according to some embodiments of the present invention.

Figures 4-8 show graphs illustrating results of processing of data from acoustic sensors according to some embodiments of the present invention.

## Detailed Description

For clarity of disclosure, and not by way of limitation, the detailed description of the invention is divided into the following subsections that describe or illustrate certain features, embodiments or applications of the present invention.

In some embodiments, the present invention provides a system for detecting, recording and analyzing aucoustic data from a pregnant mother carrying a fetus to discern cardiac data of mother and/or fetus. In some embodiments, a plurality of acoustic sensors is used to record phonocardiogram (PCG) signals, representative of cardiac activity data. The phonocardiogram signals are recordings of all the sounds made by the heart during a cardiac cycle due to, typically, for example, vibrations created by closure of the heart valves. For instance, there can be at list two sound generating events: the first when the atrioventricular valves close at the beginning of systole (SI) and the second when the aortic valve and pulmonary valve close at the end of systole (S2).

In some embodiments, the exemplary inventive system of the present invention can utilize a plurality of acoustic sensors (e.g., 2, 3, 4, 5, 6, 7, 8, 9, 10, etc.) to be positioned on and/or near the abdomen of a pregnant woman. For example, in some embodiments, the acoustic sensors are directly attached. In some embodiments, the acoustic sensors are incorporated into an article, such as, for example, a belt, a patch, and the like, and the article is worn by, or placed on, the pregnant mother. In some embodiments, the recorded acoustic signal data from the plurality of acoustic sensors is transmitted via, for example, at least one communication link, originated by at least one data transmitter resided in the article, to at least one remote server for the processing by at least one specifically programmed computer processor in accordance with the principles of the present invention. In some embodiments, the communication link is based on at least one wireless-type communication implementations (e.g., low emission Bluetooth (Bluetooth Low Energy). In some embodiments, the exemplary system of the present invention for recording, detecting and analyzing acoustic signal data includes an analog pre-processing module, an analog to digital converter/ microcontroller (ADC/MCU) module, a communications module, a smartphone module, and a cloud computing module. In some embodiments, the analog pre-processing module performs at least one function selected from the group consisting of: amplification of the recorded signals, and filtering the recorded signals. In some embodiments, the ADC/MCU module performs at least one task selected from the group consisting of: converting analog signals to digital signals, , converting the recorded signals to digital signals, compressing the data, digital filtering, and transferring the recorded PCG signals data to the transmitter. In some embodiments, the communications module transmits the recorded signals to a wireless receiver of the system of the present invention.

In some embodiments, the choice of acoustic sensors is readily determined by one of ordinary skill in the art. Factors influencing acoustic sensor choice include, but are not limited to, the sensitivity of the microphone component, the size of the acoustic sensor, the weight of the acoustic sensor, and the like. In some embodiments, the acoustic sensor is configured to compensate for the changes in sound propagation caused by the skin-air interface. Acoustic signals comprise sound waves or vibrations that propagate as a mechanical wave of pressure and displacement, through a medium such as air, water, or the body. Without intending to be limited to any particular theory, the behavior of sound propagation can be affected by the relationship between the density and pressure of the medium though which the sound wave propagates. Also, the behavior of sound propagation can be affected by the motion of the medium though which the sound wave propagates. Furthermore, the behavior of sound propagation can be affected by the viscosity of the medium though which the sound wave propagates.

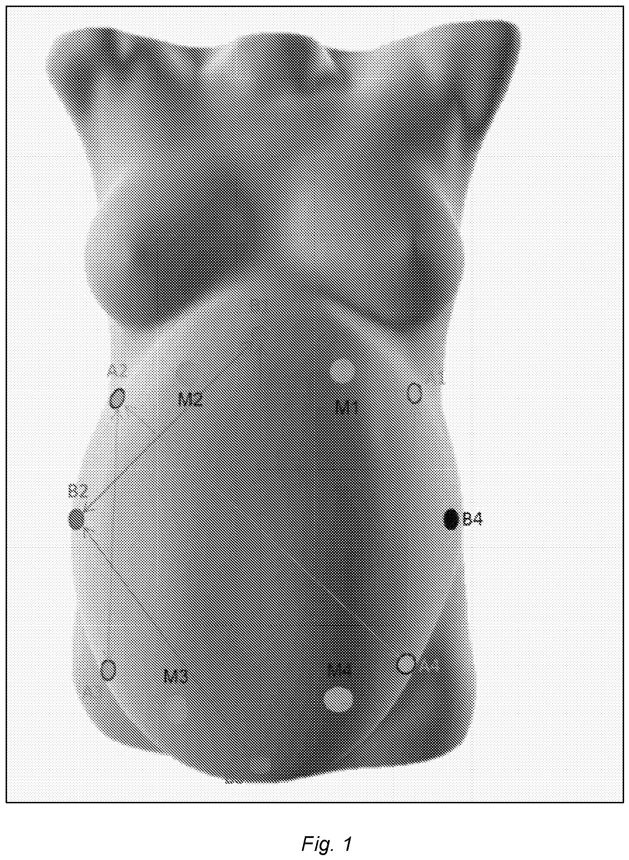

For example, as shown in Figure 1, the exemplary inventive system of the present invention utilizes a set of four acoustic sensors (M1-M4) at respective exemplary positions. In some embodiments, the positioning of acoustic sensors can varies based, at least in part, on, for example, shape of mother's stomach, the stage of the pregnancy, physiological characteristics of mother and/or fetus(es), previous acoustic and/or other types of cardio recordings (e.g., Electrocardiogram (ECG) signal recordings and analysis, etc.), and other similarly suitable data.

In some embodiments, the acoustic sensors of the present invention record the internal sound produced inside the woman with added noise from the environment. As detailed below, from these recordings the heartbeat sound of the fetus(es) and/or the mother are extracted and the heart rate of each subject is calculated.

In some embodiments, the level of detection by each acoustic sensor is independent of the other acoustic sensors (e.g, in Fig. 1, on other three acoustic sensors). Referring to Figure 1, in some embodiments, it is determined that, typically, the fetus PCG signals are detected by the acoustic sensors in locations M3 and/or M4, while the maternal PCG signals are detected by the acoustic sensors in locations M1 and/or M2. In some embodiments, the maternal PCG signals can be detected by all four sensors (M1-M4) and have to be cleaned in order to detect the fetus(es) heartbeats. In some embodiments, as detailed below, the cleaning process is performed using at least one Independent component analysis (ICA) algorithm of the present invention. For instance, in some embodiments, the inventive system of the present invention assumes that the interfering noises are audio sources which are not the fetal origin that thus are statistically independent from the fetal heart sounds.

<u>An example of an illustrative process utilized to estimate the fetus(es) and/or maternal heartbeat data in accordance with the present invention</u>

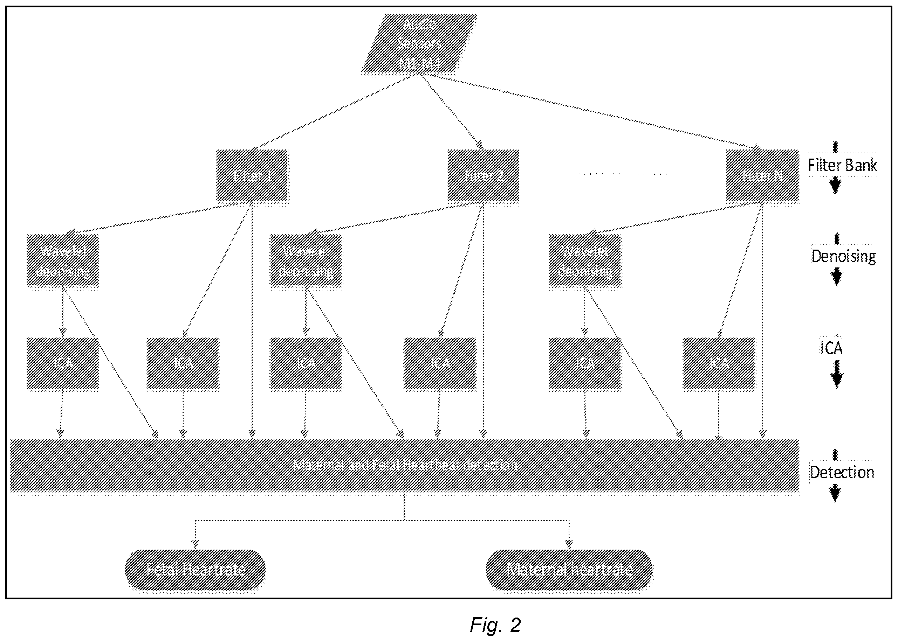

Figure 2 shows an exemplary process utilized, in some embodiments, by the exemplary inventive system of the present invention to estimate the fetus(es) and/or maternal heartbeat data (e.g., presence of heartbeats, heartbeat patterns, heartbeat frequency, ect.).

Referring to Fig. 2, the illustrative process contains at the following four stages.

<u>Stage 1: Filter Bank</u>

Referring to Fig. 2, the input is the signal data recorded by each acoustic sensor (M1-M4) shown in Fig. 1. In some embodiments, the recorded signal data from each of the four acoustic sensors is passed through, for example but not limited to, L number of bandpass filters, where each filter has a predetermine frequency range. In some embodiments, the exemplary six bandpass filters (L=6) can have the following six bandwidths:

10-45Hz

20-50Hz

25-65Hz

40-80Hz

55-95Hz

20-80Hz.

In some embodiments, there can be at least 3 bandpass filters. In some embodiments, there can be between 3-12 bandpass filters. In some embodiments, there can be between 5-10 bandpass filters. In some embodiments, there can be at least 5 bandpass filter. In some embodiments, there can be at least 7 bandpass filter.

In some embodiments, the bandwidth of a particular filter can be selected from a range of 10-100Hz. In some embodiments, the bandwidth of a particular filter can be selected from a range of 20-100Hz. In some embodiments, the bandwidth of a particular filter can be selected from a range of 30-100Hz. In some embodiments, the bandwidth of a particular filter can be selected from a range of 40-100Hz. In some embodiments, the bandwidth of a particular filter can be selected from a range of 50-100Hz. In some embodiments, the bandwidth of a particular filter can be selected from a range of 60-100Hz. In some embodiments, the bandwidth of a particular filter can be selected from a range of 70-100Hz. In some embodiments, the bandwidth of a particular filter can be selected from a range of 80-100Hz. In some embodiments, the bandwidth of a particular filter can be selected from a range of 90-100Hz.

In some embodiments, a particular filter can have a frequency range of 5-110Hz. In some embodiments, a particular filter can have a frequency range of 15-110Hz. In some embodiments, a particular filter can have a frequency range of 25-110Hz. In some embodiments, a particular filter can have a frequency range of 35-110Hz. In some embodiments, a particular filter can have a frequency range of 45-110Hz. In some embodiments, a particular filter can have a frequency range of 55-110Hz. In some embodiments, a particular filter can have a frequency range of 5-105Hz. In some embodiments, a particular filter can have a frequency range of 5-100Hz. In some embodiments, a particular filter can have a frequency range of 5-95Hz. In some embodiments, a particular filter can have a frequency range of 5-25Hz. In some embodiments, a particular filter can have a frequency range of 5-50Hz. In some embodiments, a particular filter can have a frequency range of 5-75Hz.

In some embodiments, referring to Fig. 2, a K number of outputs (filtered PCG outputs) from each of the six filters are forwarded to the second stage. For example, in some embodiments, detailed below, K is equal to, but not limited to, 4. In some embodiments, K is equal to the number of acoustic sensors (M1-M4) being used to collect the acoustic sensor data. In some embodiments, a predetermined number of outputs is forwarded to the second stage.

Stage 2: Wavelet denoising

In some embodiments, for example, one of the four filtered PCG outputs from the six bandpass filters is further subject to wavelet denoising, where such particular filtered PCG output is deconstructed by passing through a succession of low and high pass filters up to X times to form the deconstructed filtered PCG output. In some embodiments, the value of X depends on a sampling frequency which allows to achieve suitable results to meet requirements detailed below. For example, in some embodiments, the particular filtered PCG output is deconstructed by passing through a succession of low and high pass filters up to 3 times. For example, in some embodiments, the particular filtered PCG output is deconstructed by passing through a succession of low and high pass filters up to 5 times. For example, in some embodiments, the particular filtered PCG output is deconstructed by passing through a succession of low frequency pass and high frequency pass filters up to 6 times.

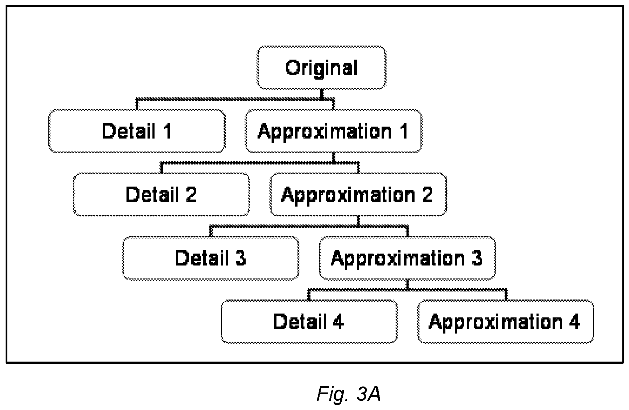

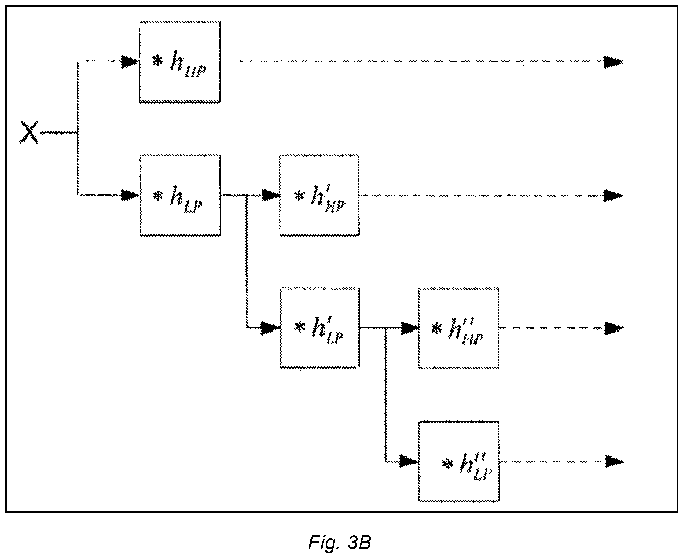

Figs. 3A and 3B show illustrative diagrams of the exemplary denoising stage. In some embodiments, the result of the denoising is the wavelet transform coefficients called approximations and details. Specifically, the deconstructed filtered PCG output can be reconstructed using the details: $D_j(n)$, $j=1,...,N$ and the approximation $A_j(n)$ to form the denoised filtered PCG output.

In some embodiments, the denoised filtered PCG output is determined based on equation 1:

$$y_r(n) = A_N(n) + \sum_{j=1}^{N} D_j(n) \qquad (1).$$

Specifically, initially, in the first step of the denoising, all details that most likely do not carry information of the heartbeat signal are set to 0, for example but not limited to : $D\_j(n)=0$, $j=1, 2, 3, 5, 6, \ldots, N$. For example, details that do not contain sound data corresponding to S1 and/or S2 phases of the cardiac contraction cycle.

Then, the remaining detail, the fourth detail, is thresholded using a threshold calculated utilizing, for example but not limited to, Stein's Unbiased Risk Estimate (SURE) method illustrated by equation 2:

$$D_4(n) = \begin{cases} 0 & \forall \ D_4(n) < TH_{SURE} \\ D_4(n) & Otherwise \end{cases} \qquad (2).$$

In some embodiments, the threshold can be calculated using any other similarly suitable method.

In some embodiments the remaining detail can be any of the details or a combination of them.

Lastly, the denoised filtered PCG output is calculated using equation (1).

Stage 3: ICA transformation

Referring to Fig. 2, (1) the filtered PCG output and (2) the denoised filtered PCG output are further transformed using at least one ICA algorithm to form (1) the transformed filtered PCG output and (2) the transformed denoised filtered PCG output, respectively. In some embodiments, the exemplary ICA algorithm is, for example but not limited to, the FAST ICA algorithm. In some embodiments, the FAST ICA algorithm is, for example, utilized in accordance with "Independent component analysis: Algorithms and applications," Hyvärinen et al., Neural Networks 13 (4-5): 411-430 (2000), whose specific descriptions are hereby incorporated herein for such specific purpose.

The term "transformation(s)" as used herein refers to linear or non-linear mathematical transformations, which may include, *inter alia,* digital filtering, mathematical linear or non-linear decomposition, mathematical optimization.

Stage 4: Detection of fetal and maternal heart beats

To summarize, at this stage, all outputs of the filterbank, denoisng, and ICA, from the previous three stages are being utilized as a S number of detection heartbeat (DH) inputs, where S is a number of all outputs of the filterbank, denoisng, and ICA stages, calculated as LxM, as determined below:

1) M filtered PCG outputs, resulted from 4 signal data inputs of the acoustic sensors MI-M4 being passed through L bandpass filters;

2) M filtered ICA transforms of the filtered PCG outputs;

3) M denoised filtered PCG outputs; and

4) M denoised filtered ICA transforms of the denoised filtered PCG outputs, where M= LxK.

In some embodiments, the exemplary inventive system of the present detection assumes that the heart beats of the mother and/or the fetus(es) can be detected in each one of these DH inputs. In some embodiments, the exemplary inventive system has no prior historical data to suggest in which of these DH inputs the heartbeat of the fetus(es) would be detected, or the heartbeat of the mother, or both, or none.

For example, in some embodiments, the L number of filters in the filter bank is 6 and the total number of inputs for stage 4 is M x L = 96.

In some embodiments, the stage 4 is further divided into four sub-stages:

Sub-stage 1: Detect all heartbeats in each of the DH inputs;

Sub-stage 2: Calculate a confidences score that describes the probability that the heartbeats detected in Step 1 are actual heartbeats and not the noise;

Sub-stage 3: Divide all the DH inputs into at least two groups:

- Group 1: fetal heartbeat outputs that contain fetal heartbeats

- Group 2: maternal heartbeat outputs that contain maternal heartbeats;

Sub-stage 4: Select from each group the most probable output that contains the corresponding heartbeat.

Sub-stage 1: Heartbeat detection

Typically, acoustic signal can contain at least some noise and heartbeats in at least some cases can be "covered" with noise or in a very noisy surrounding. In addition, typically, the heartbeat morphology can vary form one heartbeat to the next. In some embodiments, the Sub-stage 1 is carried out in at least the following number of steps.

Step 1: Beat detection

i) In some embodiments, the exemplary inventive system of the present invention calculates a slow envelope of the absolute of the Hilbert transform of the acoustic signal. For instance, exemplary inventive system of the present invention calculates this envelope by applying a moving average filter on the absolute value of the Hilbert transform. In some embodiments, the exemplary inventive system of the present invention can utilize the moving average window having a predetermined length of P. For example, in some embodiments, P is 300 milliseconds (ms). In some embodiments, P varies from 100 to 500 ms. In some embodiments, P varies from 200 to 500 ms. In some embodiments, P varies from 300 to 500 ms. In some embodiments, P varies from 400 to 500 ms.

ii) The exemplary inventive system of the present invention then determines all the peaks of the signal by identifying the zero crossings of the derivative of the signal in each respective DH input.

iii) The exemplary inventive system of the present invention then discards all peaks that are not prominent enough, based, at least in part, on the following criteria used is:

$$Select \; \forall \; peaks \; such \; that \; \frac{p(k)}{median(\{p(1),p(2),...,p(N)\})} > Thresh \; k=1..N \; where \; p(k) \; is \; the \; prominence \; of \; the \; k^{th} \; peak.$$

iv) The exemplary inventive system of the present invention then groups all peaks into two groups according to, for example, shape and size. For example, such grouping is done using a Gaussian Mixture model clustering algorithm with the following features for each peak:

- the width of each peak divided by the max peak width, and

- the height of the peak divided by its prominence.

v) The exemplary inventive system of the present invention then selects a group of peaks that has the smallest variance measure as the peaks for the next steps.

vi) The exemplary inventive system of the present invention then repeats steps i-v using an average window length R ms. In some embodiments, the average window length of 250 ms. In some embodiments, the average window length is between 100-300 ms.

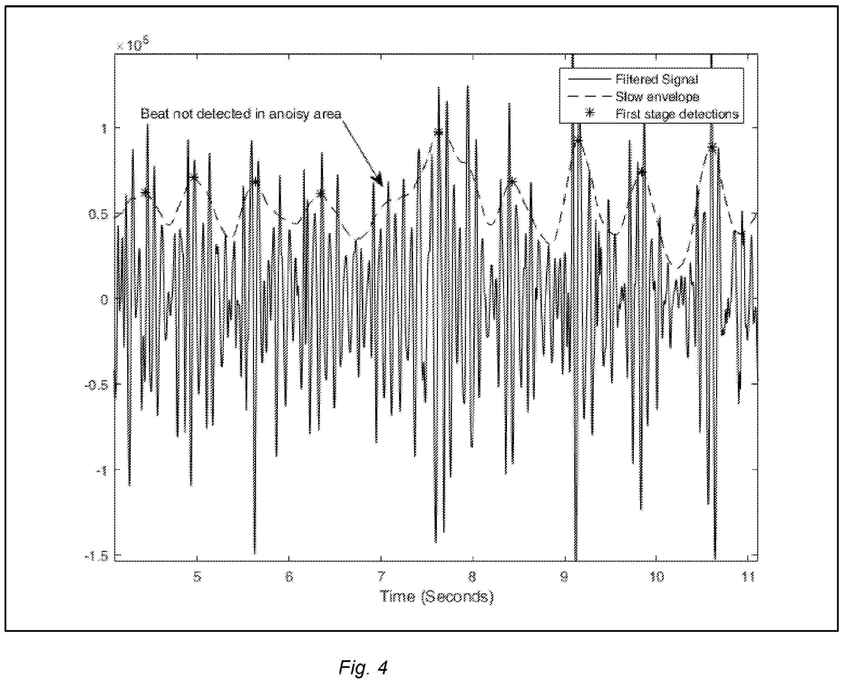

vii) The exemplary inventive system of the present invention then calculates the Excess Kurtosis measure of the distribution (an adjusted version of Pearson's kurtosis) for the locations of peaks, and selects the peak locations that provide the smallest Excess Kurtosis. Figure 4 shows the result of the detection at this step, specifically the beat located in a noisy area is not detected.

viii) From the selected peaks locations, the exemplary inventive system of the present invention then calculates an initial estimate of the average Heart Rate (HR).

Step 2 - Enhanced Detection

The exemplary inventive system of the present invention refines the detection results of Step 1 by, for example, adding missing beats and correcting the Step 1 detection locations.

i) The exemplary inventive system of the present invention calculates a fast envelope using a Q ms window in the same manner performed as detailed in item (i) of Step 1. For example, in some embodiments, Q is 100 ms. In some embodiments, Q varies between 50 -200 ms. In some embodiments, Q varies between 50 - 150 ms. In some embodiments, Q varies between 50 - 100 ms.

ii) The exemplary inventive system of the present invention then identifies peaks on the fast envelope signal and selects all prominent peaks in same manner as detailed in item (iii) of Step 1.

iii) The exemplary inventive system of the present invention then calculates all beat to beat intervals (B2B) from the detection results of Step 1, where B2B is an interval between successive beats.

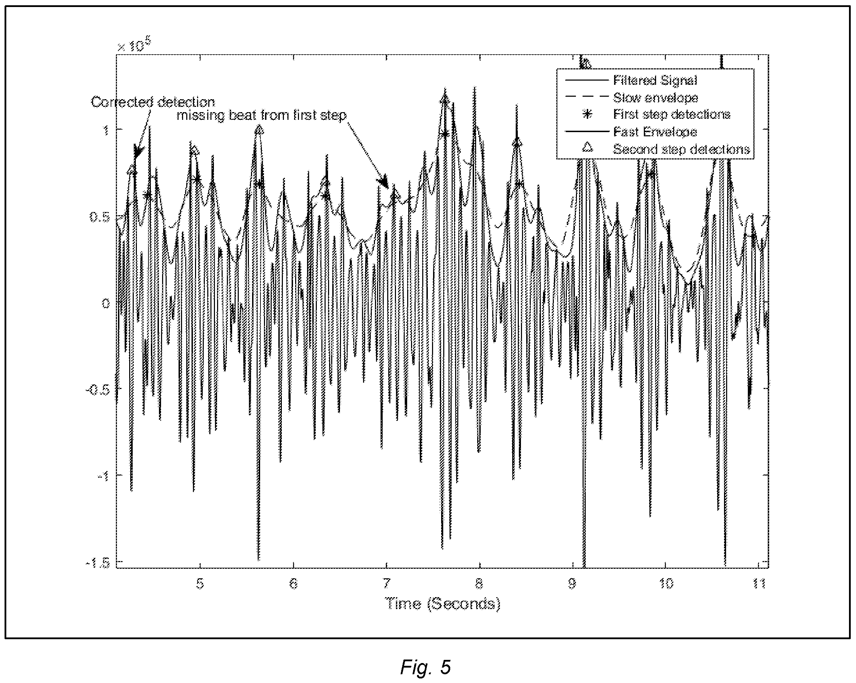

iv) Referring to Fig. 5, the exemplary inventive system of the present invention then completes missing beats which are not identified in Step 1, by reiteratively analyzing all beat to beat intervals determined in the item (iii) of Step 2 based on at least the following exemplary, but not limiting, conditions:

- if B2B(k) is higher than 1.5 x B2B(average) calculated in item (viii) of Step 1, find a peak from the fast envelope which is as close as possible to the last peak found in item (viii) of Step 1 + B2B(average).

v) The exemplary inventive system of the present invention then corrects the detection of Step 1, using the fast envelope.

In some embodiments, the exemplary inventive system of the present invention performs Step 2 to the extent necessary to minimize the beat to beat variance.

Sub-stage 2: Calculate a confidence score for the heartbeat detection

Typically, a beat-by-beat heart rate is a physiological signal which cannot change too fast and usually follows a baseline that defines an average heart rate. In some embodiments, the exemplary inventive system of the present invention utilizes the average heart rate as the base for calculating the confidence score. For each detection of heart beats (for example but not limited to 96 of them, obtained at the end of Sub-Stage 1 from 96 DH inputs), the exemplary inventive system of the present invention calculates the confidence score in accordance with the following steps.

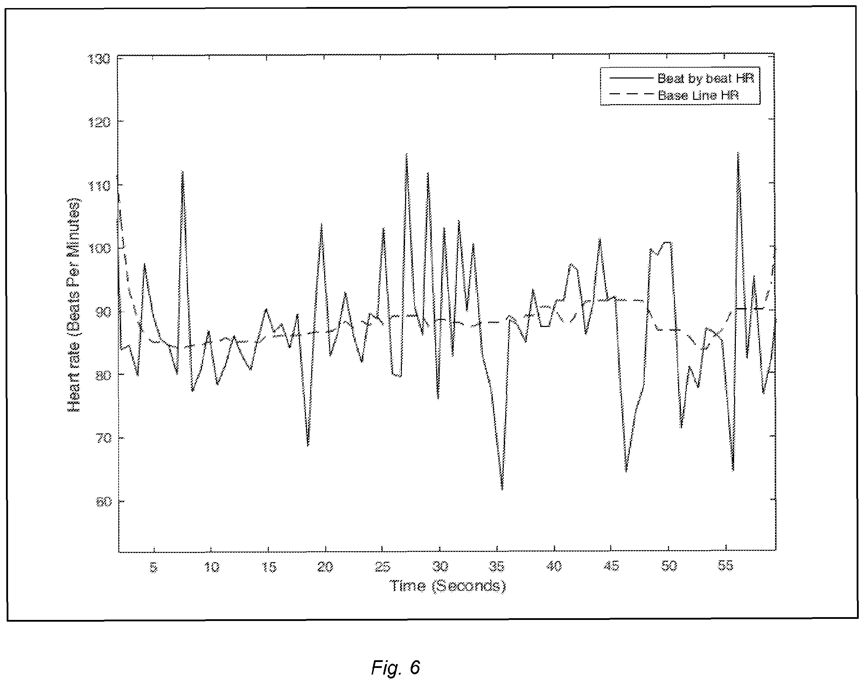

i) The exemplary inventive system of the present invention generates a beat-by-beat heartbeat graph (shown in Fig. 6) based on the locations of the beats.

ii) The exemplary inventive system of the present invention then estimates the heart rate for the whole signal (HRestim), by calculating a histogram of all the beat-by-beat heart rates, where the estimated heart rate for the whole signal is at the maximum of the histogram.

iii) The exemplary inventive system of the present invention then estimates a base line for the beat-by-beat heartbeat graph (Fig. 6) using a median filter (e.g., order 20, etc.).

iv) The exemplary inventive system of the present invention then calculates the confidence score based on the following equation (3):

$$\text{Score} = \sqrt{\frac{\text{MfRR}^2 + \text{SfRR}^2 + \text{OvMP}^2}{3}}, \qquad (3),$$

where $MfRR = \frac{\sum_{n=1}^{N} |HR(n) - HRestim|}{N}$,   SfRR   =   std(|HR(n)   -   HRestim|),   OvMP   =

$$\tfrac{1}{N} \sum \; 1_{HR} \, , \; 1_{HR} = \begin{cases} 1, & |HR(n) - HRestim| < HRestim \times 0.15 \\ 0, & otherwise \end{cases} \; .$$

Sub-stage 3: Group all heartbeat detections into 2 groups: Maternal and Fetal

Based on the Stages 1-3 and Sub-stages 1-2, The exemplary inventive system of the present invention generates S beat-by-beat heartbeat graphs, which are vectors representative of the DH inputs obtained from Stages 1-3 (DH input vectors). For example, in case of 96 DH inputs, 96 beat-by-beat heartbeat graphs is generated. Some of the DH input vectors represent maternal heartbeat and some represent the fetal heartbeat.

In some embodiments, the exemplary inventive system of the present invention can utilize, for example, other contemporaneous maternal heartbeat data about the maternal heartbeat which has been separately determined based on data collected from non-acoustic sensor(s)/equipment (e.g., ECG data) to group all DH input vectors that are highly correlated with the other contemporaneous maternal heartbeat data as candidates for maternal heart rate detection and the remaining DH input vectors are grouped as fetal heartbeat candidates.

In some embodiments, when the other contemporaneous maternal heartbeat data is not available, the exemplary inventive system of the present invention can group the DH input vectors, by clustering the estimated heart rate of each DH input vector according to its value. In some embodiments, the exemplary inventive system of the present invention then designates the DH input vectors with a higher average heartbeat rate into the fetal heartbeat group.

Sub-stage 4: Select the fetal and maternal best detections

In some embodiments, based on the best confidence score, the exemplary inventive system of the present invention selects the best representative maternal DH input vector as the best detection of the maternal heartbeat rate from the group of the maternal heartbeat DH input vectors identified in Sub-stage 3.

In some embodiments, based on the best confidence score, the exemplary inventive system of the present invention selects the best representative fetal DH input vector as the best detection of the fetal heartbeat rate from the group of the fetal DH input vectors identified in Sub-stage 3.

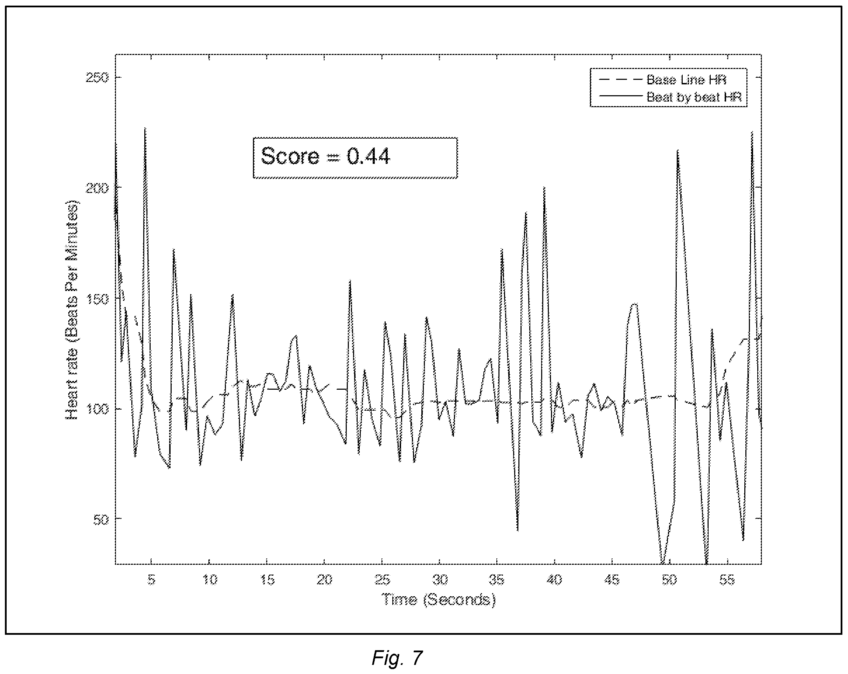

Fig. 7 shows an illustrative example a beat-by-beat heartbeat graph representative of an exemplary DH input vector with a low confidence score.

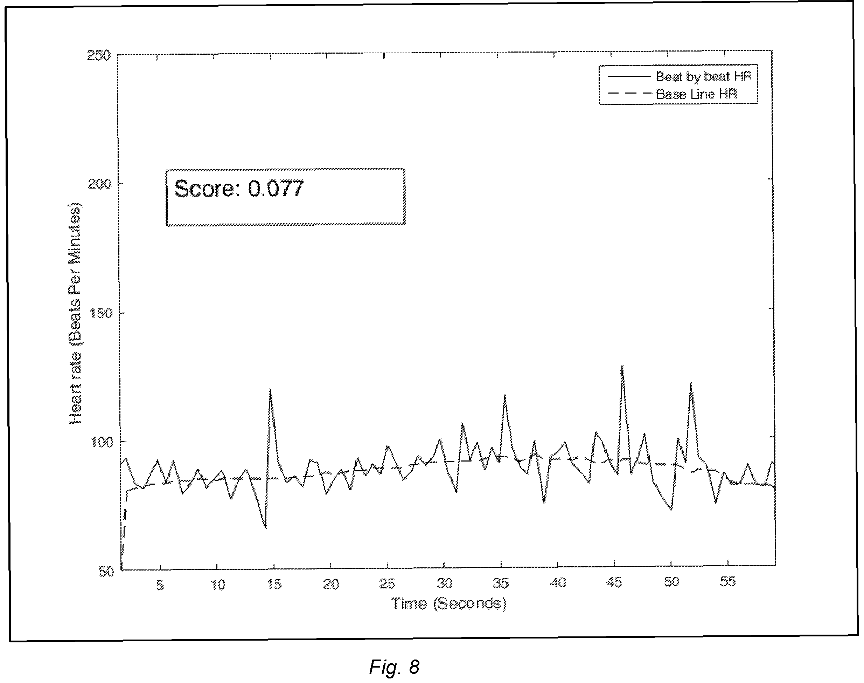

Fig. 8 shows an illustrative example a beat-by-beat heartbeat graph representative of an exemplary DH input vector with a high confidence score.

In some embodiments, the instant invention is directed to a computer-implemented method which includes at least the steps of: receiving, by at least one computer processor executing specific programmable instructions configured for the method, a plurality of Phonocardiogram (PCG) signals data inputs from a plurality of acoustic sensors; digital signal filtering, by the at least one computer processor, utilizing a plurality of bandpass filters, the plurality of PCG signals data inputs to form a plurality of filtered PCG outputs, where the plurality of bandpass filters includes a L number of bandpass filters, where each bandpass filter outputs a K number of filtered PCG outputs; wavelet denoising, by the at least one computer processor, a first subset of filtered PCG outputs of the plurality of filtered PCG outputs to form a M number of denoised filtered PCG outputs, where M is equal to L multiply by K; transforming, by the at least one computer processor, utilizing an Independent-Component-Analysis (ICA), a second subset of filtered PCG outputs of the plurality of filtered PCG outputs to form the M number of filtered ICA transforms; transforming, by the at least one computer processor, utilizing the Independent-Component-Analysis (ICA), a first portion of the second subset of denoised filtered PCG outputs to form the M number of filtered denoised filtered ICA transforms; compiling, by the at least one computer processor, a S number of a plurality of detection heartbeat (DH) inputs, including: i) the M number of filtered PCG outputs, ii) the M number of denoised filtered PCG outputs, iii) the M number of filtered ICA transforms, and iv) the M number of denoised filtered ICA transforms; detecting, by the at least one computer processor, beat locations of beats in each of DH inputs; calculating, by the at least one computer processor, a confidence score that describes a probability that the beats in each DH input of the plurality of DH inputs represent actual heartbeats and not a noise; dividing, by the at least one computer processor, the plurality of DH inputs into at least two groups: i) a first group of DH inputs containing fetal heartbeats, ii) a second group of DH inputs containing maternal heartbeats selecting, by the at least one computer processor, from the first group of DH inputs, at least one particular fetal DH input that contains the fetal heartbeat based on a first confidence score of the at least one particular fetal DH input; and selecting, by the at least one computer processor, from the second group of DH inputs, at least one particular maternal DH input that contains the maternal heartbeat, based on a second confidence score of the at least one particular maternal DH input.

In some embodiments, where the wavelet denoising includes at least: deconstructing, by the at least one computer processor, each filtered PCG output to generate a plurality of transform coefficients, by irrelatively passing each filtered PCG output through a succession of low and high pass filters; identifying, by the at least one computer processor, a subset of heartbeat-carrying transform coefficients from the plurality of transform coefficients, reconstructing, by the at least one computer processor, the subset of heartbeat-carrying transform coefficients to form the M number of denoised filtered PCG outputs.

In some embodiments, K is equal to a number of the plurality of acoustic sensors, and L is equal to 6.

In some embodiments, each filter of the plurality of bandpass filters has a bandwidth of 10-100Hz and a frequency range of 5-110HZ.

In some embodiments, the detecting of the beat locations of the beats in each of DH inputs includes at least: calculating, by the at least one computer processor, a predetermined transform filter; iteratively repeating, by the at least one computer processor, based on a predetermined average window length of the predetermined transform filter: identifying, in each DH input, a subset of peaks having a predetermined shape and a predetermined size, and selecting a group of peaks from the subset of peaks, where the group of peaks has the smallest variance measure; calculating, by the at least one computer processor, initial locations of the beats.

In some embodiments, the calculating the confidence score includes at least: generating, by the at least one computer processor, a beat-by-beat heartbeat graph for each DH input based on the beat locations.

In some embodiments, the dividing the plurality of DH inputs into the first group of DH inputs containing fetal heartbeats and the second group of DH inputs containing maternal heartbeats includes at least: clustering, by the at least one computer processor, the beats of each DH input according to each beat value; and assigning, by the at least one computer processor, a particular DH input having a higher average beat rate into the first group of DH inputs containing fetal heartbeats.

In some embodiments, the present invention is directed to a specifically programmed computer system, including at least the following components: at least one specialized computer machine, including: a non-transient memory, electronically storing particular computer executable program code; and at least one computer processor which, when executing the particular program code, becomes a specifically programmed computing processor that is configured to at least perform the following operations: receiving a plurality of Phonocardiogram (PCG) signals data inputs from a plurality of acoustic sensors; digital signal filtering, utilizing a plurality of bandpass filters, the plurality of PCG signals data inputs to form a plurality of filtered PCG outputs, where the plurality of bandpass filters includes a L number of bandpass filters, where each bandpass filter outputs a K number of filtered PCG outputs; wavelet denoising a first subset of filtered PCG outputs of the plurality of filtered PCG outputs to form a M number of denoised filtered PCG outputs, where M is equal to L multiply by K; transforming, utilizing an Independent-Component-Analysis (ICA), a second subset of filtered PCG outputs of the plurality of filtered PCG outputs to form the M number of filtered ICA transforms; transforming, utilizing the Independent-Component-Analysis (ICA), a first portion of the second subset of denoised filtered PCG outputs to form the M number of denoised filtered ICA transforms; compiling a S number of a plurality of detection heartbeat (DH) inputs, including: i) the M number of filtered PCG outputs, ii) the M number of denoised filtered PCG outputs, iii) the M number of filtered ICA transforms, and iv) the M number of denoised filtered ICA transforms; detecting beat locations of beats in each of DH inputs; calculating a confidence score that describes a probability that the beats in each DH input of the plurality of DH inputs represent actual heartbeats and not a noise; dividing the plurality of DH inputs into at least two groups: i) a first group of DH inputs containing fetal heartbeats, ii) a second group of DH inputs containing maternal heartbeats; selecting, from the first group of DH inputs, at least one particular fetal DH input that contains the fetal heartbeat based on a first confidence score of the at least one particular fetal DH input; and selecting, from the second group of DH inputs, at least one particular maternal DH input that contains the maternal heartbeat, based on a second confidence score of the at least one particular maternal DH input.

## Claims

1. A computer-implemented method, comprising:

   - receiving, by at least one computer processor executing specific programmable instructions configured for the method, a plurality of Phonocardiogram (PCG) signals data inputs from a plurality of acoustic sensors;
   - digital signal filtering, by the at least one computer processor, utilizing a plurality of bandpass filters, the plurality of PCG signals data inputs to form a plurality of filtered PCG outputs, wherein the plurality of bandpass filters comprises a L number of bandpass filters, wherein each bandpass filter outputs a K number of filtered PCG outputs;
   - wavelet denoising, by the at least one computer processor, a first subset of filtered PCG outputs of the plurality of filtered PCG outputs to form a M number of denoised filtered PCG outputs, wherein M is equal to L multiply by K, wherein the wavelet denoising comprises:

     - deconstructing, by the at least one computer processor, each filtered PCG output to generate a plurality of transform coefficients, by irrelatively passing each filtered PCG output through a succession of low and high pass filters,

- identifying, by the at least one computer processor, a subset of heartbeat-carrying transform coefficients from the plurality of transform coefficients, and

- reconstructing, by the at least one computer processor, the subset of heartbeat-carrying transform coefficients to form the M number of denoised filtered PCG outputs;

- transforming, by the at least one computer processor, utilizing an Independent-Component-Analysis (ICA), a second subset of filtered PCG outputs of the plurality of filtered PCG outputs to form the M number of filtered ICA transforms;

- transforming, by the at least one computer processor, utilizing the Independent-Component-Analysis (ICA), a first portion of the second subset of denoised filtered PCG outputs to form the M number of denoised filtered ICA transforms;

- compiling, by the at least one computer processor, a S number of a plurality of detection heartbeat (DH) inputs, comprising:

  i) the M number of filtered PCG outputs,
  ii) the M number of the denoised filtered PCG outputs,
  iii) the M number of the filtered ICA transforms, and
  iv) the M number of the denoised filtered ICA transforms;

- detecting, by the at least one computer processor, beat locations of beats in each of DH inputs;

- calculating, by the at least one computer processor, a confidence score that describes a probability that the beats in each DH input of the plurality of DH inputs represent actual heartbeats and not a noise;

- dividing, by the at least one computer processor, the plurality of DH inputs into at least two groups:

  i) a first group of DH inputs containing fetal heartbeats,
  ii) a second group of DH inputs containing maternal heartbeats;

- selecting, by the at least one computer processor, from the first group of DH inputs, at least one particular fetal DH input that contains the fetal heartbeat based on a first confidence score of the at least one particular fetal DH input; and

- selecting, by the at least one computer processor, from the second group of DH inputs, at least one particular maternal DH input that contains the maternal heartbeat, based on a second confidence score of the at least one particular maternal DH input.

2. The computer-implemented method of claim 1,
wherein K is equal to a number of the plurality of acoustic sensors, and L is equal to 6.

3. The computer-implemented method of claim 1 or 2,
wherein each filter of the plurality of bandpass filters has a bandwidth of 10-100 Hz and a frequency range of 5-110 HZ.

4. The computer-implemented method of one of claims 1 to 3,
wherein the detecting of the beat locations of the beats in each of DH inputs, comprising:

- calculating, by the at least one computer processor, a predetermined transform filter;
- iteratively repeating, by the at least one computer processor, based on a predetermined average window length of the predetermined transform filter:

  - identifying, in each DH input, a subset of peaks having a predetermined shape and a predetermined size, and
  - selecting a group of peaks from the subset of peaks, wherein the group of peaks has the smallest variance measure;
  - calculating, by the at least one computer processor, initial locations of the beats.

5. The computer-implemented method of one of claims 1 to 4,
wherein the calculating the confidence score, comprising:

- generating, by the at least one computer processor, a beat-by-beat heartbeat graph for each DH input based on the beat locations.

6. The computer-implemented method of one of claims 1 to 5,
wherein the dividing the plurality of DH inputs into the first group of DH inputs containing fetal heartbeats and the second group of DH inputs containing maternal heartbeats, comprising:

- clustering, by the at least one computer processor, the beats of each DH input according to each beat value; and
- assigning, by the at least one computer processor, a particular DH input having a higher average beat rate into the first group of DH inputs containing fetal heartbeats.

7. A specifically programmed computer system, comprising:

- at least one specialized computer machine, comprising:

- a non-transient memory, electronically storing particular computer executable program code; and
- at least one computer processor which, when executing the particular program code, becomes a specifically programmed computing processor that is configured to at least perform the following operations:

- receiving a plurality of Phonocardiogram (PCG) signals data inputs from a plurality of acoustic sensors;
- digital signal filtering, utilizing a plurality of bandpass filters, the plurality of PCG signals data inputs to form a plurality of filtered PCG outputs, wherein the plurality of bandpass filters comprises a L number of bandpass filters, wherein each bandpass filter outputs a K number of filtered PCG outputs;
- wavelet denoising a first subset of filtered PCG outputs of the plurality of filtered PCG outputs to form a M number of denoised filtered PCG outputs, wherein M is equal to L multiply by K, wherein the wavelet denoising comprises:

- deconstructing each filtered PCG output to generate a plurality of transform coefficients, by irrelatively passing each filtered PCG output through a succession of low and high pass filters,
- identifying a subset of heartbeat-carrying transform coefficients from the plurality of transform coefficients, and
- reconstructing the subset of heartbeat-carrying transform coefficients to form the M number of denoised filtered PCG outputs;
- transforming, utilizing an Independent-Component-Analysis (ICA), a second subset of filtered PCG outputs of the plurality of filtered PCG outputs to form the M number of filtered ICA transforms;
- transforming, utilizing the Independent-Component-Analysis (ICA), a first portion of the second subset of denoised filtered PCG outputs to form the M number of denoised filtered ICA transforms;
- compiling a S number of a plurality of detection heartbeat (DH) inputs, comprising:

i) the M number of filtered PCG outputs,
ii) the M number of denoised filtered PCG outputs,
iii) the M number of filtered ICA transforms, and
iv) the M number of denoised filtered ICA transforms;

- detecting beat locations of beats in each of DH inputs;
- calculating a confidence score that describes a probability that the beats in each DH input of the plurality of DH inputs represent actual heartbeats and not a noise;
- dividing the plurality of DH inputs into at least two groups:

i) a first group of DH inputs containing fetal heartbeats,
ii) a second group of DH inputs containing maternal heartbeats;

- selecting, from the first group of DH inputs, at least one particular fetal DH input that contains the fetal heartbeat based on a first confidence score of the at least one particular fetal DH input; and
- selecting, from the second group of DH inputs, at least one particular maternal DH input that contains the maternal heartbeat, based on a second confidence score of the at least one particular maternal DH input.

8. The specifically programmed computer system of claim 7,

wherein K is equal to a number of the plurality of acoustic sensors, and L is equal to 6.

9. The specifically programmed computer system of claim 7 or 8,
wherein each filter of the plurality of bandpass filters has a bandwidth of 10-100 Hz and a frequency range of 5-110 HZ.

10. The specifically programmed computer system of one of claims 7 to 9, wherein the detecting of the beat locations of the beats in each of DH inputs, comprising:

- calculating a predetermined transform filter;
- iteratively repeating, based on a predetermined average window length of the predetermined transform filter:

    - identifying, in each DH input, a subset of peaks having a predetermined shape and a predetermined size, and
    - selecting a group of peaks from the subset of peaks, wherein the group of peaks has the smallest variance measure;
- calculating initial locations of the beats.

11. The specifically programmed computer system of one of claims 7 to 10, wherein the calculating the confidence score, comprising:

- generating a beat-by-beat heartbeat graph for each DH input based on the beat locations.

12. The specifically programmed computer system of one of claims 7 to 11, wherein the dividing the plurality of DH inputs into the first group of DH inputs containing fetal heartbeats and the second group of DH inputs containing maternal heartbeats, comprising:

- clustering the beats of each DH input according to each beat value; and
- assigning a particular DH input having a higher average beat rate into the first group of DH inputs containing fetal heartbeats.

**Patentansprüche**

1. Computerimplementierte Methode, welche Folgendes umfasst:

- Empfangen einer Vielzahl von Inputs von Signaldaten aus einem Phonokardiogramm (PKG), durch mindestens einen Computerprozessor, der spezifische programmierbare, für die Methode konfigurierte Anweisungen ausführt;
- digitales Signalfiltern durch den mindestens einen Computerprozessor unter Einsatz einer Vielzahl von Bandpassfiltern, wobei die Vielzahl von PKG-Signaldaten-Inputs eine Vielzahl gefilterter PKG-Outputs bilden, wobei die Vielzahl von Bandpassfiltern eine Anzahl L von Bandpassfiltern enthält, wobei jeder Bandpassfilter einer Anzahl K gefilterter PKG-Outputs ausgibt;
- Wavelet-Entrauschung einer ersten Teilmenge gefilterter PKG-Outputs aus der Vielzahl gefilterter gefilterter PKG-Outputs durch den mindestens einen Computerprozessor, um eine Anzahl M entrauschter gefilterter PKG-Outputs zu bilden, wobei M gleich L multipliziert mit K ist, wobei die Wavelet-Entrauschung Folgendes umfasst:

    - Dekonstruieren jedes gefilterten PKG-Outputs durch den mindestens einen Computerprozessor, um eine Vielzahl von Transformationskoeffizienten zu generieren, indem jeder gefilterte PKG-Ouput ohne Zusammenhang durch eine aufeinanderfolgende Reihe von Tiefpass- und Hochpassfiltern hindurchgeführt wird,
    - Ermitteln einer Teilmenge herzschlagübertragender Transformationskoeffizienten aus der Vielzahl von Transformationskoeffizienten durch den mindestens einen Computerprozessor, und
    - Rekonstruieren der Teilmenge herzschlagübertragender Transformationskoeffzienten durch den mindestens einen Computerprozessor, um die Anzahl M entrauschter gefilterter PKG-Outputs zu bilden;

- Transformieren einer zweiten Teilmenge gefilterter PKG-Outputs aus der Vielzahl gefilterter PKG-Outputs durch den mindestens einen Computerprozessor unter Einsatz einer Analyse unabhängiger Komponenten (ICA), um die Anzahl M gefilterter ICA-Transformationen zu bilden;
- Transformieren eines ersten Teils der zweiten Teilmenge entrauschter gefilterter ICA-Transformationen durch den mindestens einen Computerprozessor unter Einsatz der Analyse unabhängiger Komponenten (I-CA), um die Anzahl M gefilterter ICA-Transformationen zu bilden;
- Kompilieren einer Anzahl S aus einer Vielzahl von Inputs detektierter Herzschläge (DH) durch den mindestens einen Computerprozessor, einschließlich:

    i) der Anzahl M gefilterter PKG-Outputs,
    ii) der Anzahl M der entrauschten gefilterten PKG-Outputs,
    iii) der Anzahl M der gefilterten ICA-Transformationen, und
    iv) der Anzahl M der entrauschten gefilterten ICA-Transformationen;

- Erkennen von Schlag-Positionen von Schlägen in jedem der DH-Inputs durch den mindestens einen Computerprozessor;
- Berechnen eines Vertrauenswerts durch den mindestens einen Computerprozessor, wobei der Vertrauenswert eine Wahrscheinlichkeit beschreibt, dass die Schläge in jedem DH-Input aus der Vielzahl von DH-Inputs tatsächlich Herzschläge und kein Rauschen darstellen;
- Unterteilen der Vielzahl von DH-Inputs durch den mindestens einen Computerprozessor in mindestens zwei Gruppen:

    i) eine erste, fötale Herzschläge enthaltende Gruppe von DH-Inputs,
    ii) eine zweite, mütterliche Herzschläge enthaltende Gruppe von DH-Inputs;

- Selektieren von mindestens einem besonderen fötalen DH-Inputs aus der ersten DH-Input-Gruppe durch den mindestens einen Computerprozessor, wobei der besondere fötale DH-Input den auf einem ersten Vertrauenswert des mindestens einen besonderen fötalen DH-Inputs basierenden fötalen Herzschlag enthält; und
- Selektieren von mindestens einem besonderen mütterlichen DH-Input aus der zweiten DH-Input-Gruppe durch den mindestens einen Computerprozessor, wobei der besondere mütterliche DH-Input den auf einem zweiten Vertrauenswert des mindestens einen besonderen mütterlichen DH-Inputs basierenden mütterlichen Herzschlag enthält.

2. Computerimplementierte Methode gemäß Anspruch 1,
wobei K gleich einer Anzahl aus der Vielzahl akustischer Sensoren ist, und L gleich 6 ist.

3. Computerimplementierte Methode gemäß Anspruch 1 oder 2,
wobei jeder Filter aus der Vielzahl von Bandpassfiltern eine Bandbreite von 10-100 Hz und einen Frequenzbereich von 5-110 Hz hat.

4. Computerimplementierte Methode gemäß Anspruch 1 bis 3,
wobei die Erkennung der Schlag-Positionen der Schläge in jedem der DH-Inputs Folgendes umfasst:

- Berechnen eines vorbestimmten Transformationsfilters durch den mindestens einen Computerprozessor;
- iteratives Wiederholen basierend auf einer vorbestimmten durchschnittlichen Fensterlänge des vorbestimmten Transformationsfilters durch den mindestens einen Computerprozessor:
- wodurch in jedem DH-Input die Erkennung einer Teilmenge von Peaks mit einer vorbestimmten Form und einer vorbestimmten Größe ermittelt wird, und
- wodurch eine Gruppe von Peaks aus der Teilmenge von Peaks selektiert wird, wobei die Gruppe von Peaks die kleinste Varianzmessung aufweist;
- wodurch anfängliche Positionen der Schläge durch den mindestens einen Computerprozessor berechnet werden.

5. Computerimplementierte Methode gemäß Anspruch 1 bis 4,
wobei die Berechnung des Vertrauenswerts Folgendes umfasst:

- die Generierung eines Schlag-für-Schlag Herzschlag-Graphen für jeden auf den Schlag-Positionen basierenden DH-Input.

6. Computerimplementierte Methode gemäß Anspruch 1 bis 5,
wobei die Unterteilung der Vielzahl von DH-Inputs in die erste, fötale Herzschläge enthaltende Gruppe von DH-Inputs und die zweite Gruppe, mütterliche Herzschläge enthaltende Gruppe von DH-Inputs Folgendes umfasst:

- das Clustern der Schläge jedes DH-Inputs gemäß jedem Schlagwert durch den mindestens einen Computerprozessor; und

- die Zuweisung eines besonderen DH-Inputs mit einer höheren durchschnittlichen Schlaggeschwindigkeit zur ersten, fötale Herzschläge enthaltenden Gruppe von DH-Inputs durch den mindestens einen Computerprozessor.

7. Spezifisch programmiertes Computersystem, welches Folgendes umfasst:

- mindestens eine spezialisierte Computermaschine, welche Folgendes umfasst:

- einen nicht-flüchtigen Speicher, welcher einen bestimmten computerausführbaren Programmcode elektronisch speichert; und
- mindestens einen Computerprozessor, welcher bei der Ausführung des besonderen Programmcodes ein spezifisch programmierter Computing-Prozessor wird, der dazu konfiguriert ist, mindestens die nachstehenden Operationen durchzuführen:

- Empfangen einer Vielzahl von aus Phonokardiogramm-Signalen [PKG-Signale] bestehenden Dateninputs aus einer Vielzahl akustischer Sensoren;
- Digitales Signalfiltern der Vielzahl von PKG-Signaldaten-Inputs unter Einsatz einer Vielzahl von Bandpassfiltern, um eine Vielzahl gefilterter PKG-Outputs zu bilden, wobei die Vielzahl von Bandpassfilter eine Anzahl L von Bandpassfiltern umfasst, wobei jeder Bandpassfilter eine Anzahl K gefilterter PKG-Outputs ausgibt;
- Wavelet-Entrauschung einer ersten Teilmenge gefilterter PKG-Outputs aus der Vielzahl gefilterter PKG-Outputs, um eine Anzahl M entrauschter gefilterter PKG-Outputs zu bilden, wobei M gleich L multipliziert mit K ist, und wobei die Wavelet-Entrauschung Folgendes umfasst:

- Dekonstruieren jedes gefilterten PKG-Outputs zur Generierung einer Vielzahl von Transformationskoeffizienten, indem jeder gefilterte PKG-Output ohne Zusammenhang durch eine aufeinanderfolgende Reihe von Tief- und Hochpassfiltern geführt wird,
- Erkennen einer Teilmenge herzschlagübertragender Transformationskoeffizienten aus der Vielzahl von Transformationskoeffizienten, und
- Rekonstruieren der Teilmenge herzschlagübertragender Transformationskoeffizienten, um eine Anzahl M entrauschter gefilterter PKG-Outputs zu bilden;

- Transformieren einer zweiten Teilmenge gefilterter PKG-Outputs aus der Vielzahl gefilterter PKG-Outputs unter Einsatz der Analyse unabhängiger Komponenten (ICA), um die Anzahl M gefilterter ICA-Transformationen zu bilden,
- Transformieren eines ersten Teils der zweiten Teilmenge entrauschter gefilterter PKG-Outputs unter Einsatz einer Analyse unabhängiger Komponenten (ICA), um die Anzahl M entrauschter gefilterter ICA-Transformationen zu bilden;
- Kompilieren einer Anzahl S einer Vielzahl von aus detektierten Herzschlägen (DH) bestehenden Inputs, welche Folgendes umfasst:

i) die Anzahl M gefilterter PKG-Outputs,
ii) die Anzahl M entrauschter gefilterter PKG-Outputs,
iii) die Anzahl M gefilterter ICA-Transformationen, und
iv) die Anzahl M entrauschter gefilterter ICA-Transformationen;

- Erkennen von Herzschlag-Positionen von Schlägen in jedem der DH-Inputs;
- Berechnen eines Vertrauenswert, welcher eine Wahrscheinlichkeit beschreibt, dass die in jedem aus der Vielzahl von DH-Inputs enthaltenen Schläge Herzschläge und kein Rauschen darstellen;
- Unterteilen der Vielzahl von DH-Inputs in mindestens zwei Gruppen:

i) eine erste, fötale Herzschläge enthaltende Gruppe von DH-Inputs,
ii) eine zweite, mütterliche Herzschläge enthaltende Gruppe von DH-Inputs;

- Selektieren von mindestens einem besonderen fötalen DH-Input aus der ersten Gruppe von DH-Inputs, welcher die auf einem ersten Vertrauenswert basierenden fötalen Herzschläge des mindestens einen fötalen DH-Inputs enthält; und

- Selektieren von mindestens einem besonderen mütterlichen DH-Input aus der zweiten Gruppe von DH-Inputs, welcher die auf einem zweiten Vertrauenswert basierenden mütterlichen Herzschläge des mindestens einen fötalen DH-Inputs enthält.

8. Spezifisch programmiertes Computersystem gemäß Anspruch 7, wobei K gleich einer Anzahl aus der Vielzahl akustischer Sensoren ist, und L gleich 6 ist.

9. Spezifisch programmiertes Computersystem gemäß Anspruch 7 oder 8, wobei jeder Filter aus der Vielzahl von Bandpassfiltern eine Bandbreite von 10-100 Hz und einen Frequenzbereich von 5-110 Hz aufweist.

10. Spezifisch programmiertes Computersystem gemäß den Ansprüchen 7 bis 9,
wobei das Erkennen der Schlag-Positionen der Schläge in jedem der DH-Inputs Folgendes umfasst:

- Berechnung eines vorbestimmten Transformationsfilters;
- iteratives Wiederholen basierend auf einer vorbestimmten durchschnittlichen Fensterlänge des vorbestimmten Transformationsfilters:

- wodurch in jedem DH-Input eine Teilmenge von Peaks mit einer vorbestimmten Form und einer vorbestimmten Größe ermittelt wird, und
- wodurch eine Gruppe von Peaks aus der Teilmenge von Peaks selektiert wird, wobei die Gruppe von Peaks die kleinste Varianzmessung aufweist;
- Berechnen anfänglicher Positionen der Schläge.

11. Spezifisch programmiertes Computersystem gemäß den Ansprüchen 7 bis 10,
wobei die Berechnung des Vertrauenswerts Folgendes umfasst:

- die Generierung eines Schlag-für-Schlag Herzschlag-Graphen für jeden DH-Input basierend auf den Schlag-Positionen.

12. Spezifisch programmiertes Computersystem gemäß den Ansprüchen 7 bis 11,
wobei die Unterteilung der Vielzahl von DH-Inputs in die erste, fötale Herzschläge enthaltende Gruppe von DH-Inputs und die zweite, mütterliche Herzschläge enthaltende Gruppe von DH-Inputs Folgendes umfasst:

- das Clustern der Schläge jedes DH-Inputs gemäß jedem Schlagwert; und
- die Zuweisung eines besonderen DH-Inputs mit einer höheren durchschnittlichen Schlaggeschwindigkeit zur ersten, fötale Herzschläge enthaltenden Gruppe von DH-Inputs.

**Revendications**

1. Procédé mis en œuvre par ordinateur, comprenant les étapes consistant à :

- recevoir, par au moins un processeur d'ordinateur exécutant des instructions programmables spécifiques configurées pour le procédé, une pluralité d'entrées de données de signaux de phonocardiogramme (PCG) provenant d'une pluralité de capteurs acoustiques ;
- filtrer numériquement, par ledit au moins un processeur d'ordinateur, en utilisant une pluralité de filtres passe-bande, la pluralité de signaux d'entrée de données de signaux PCG pour former une pluralité de sorties PCG filtrées, la pluralité de filtres passe-bande comprenant un nombre L de filtres passe-bande, chaque filtre passe-bande délivrant un nombre K de sorties PCG filtrées ;
- débruiter par ondelettes, par ledit au moins un processeur d'ordinateur, un premier sous-ensemble de sorties PCG filtrées de la pluralité de sorties PCG filtrées pour former un nombre M de sorties PCG filtrées débruitées, M étant égal à L multiplié par K, le débruitage par ondelettes consistant à :

- déconstruire, par ledit au moins un processeur d'ordinateur, chaque sortie PCG filtrée pour générer une pluralité de coefficients de transformation, en faisant passer non relativement chaque sortie PCG filtrée par une succession de filtres passe-bas et passe-haut,
- identifier, par ledit au moins un processeur d'ordinateur, un sous-ensemble de coefficients de transformation porteurs de battements cardiaques parmi la pluralité de coefficients de transformation, et
- reconstruire, par ledit au moins un processeur d'ordinateur, le sous-ensemble de coefficients de transformation porteurs de battements cardiaques pour former le nombre M de sorties PCG filtrées débruitées ;

- transformer, par ledit au moins un processeur d'ordinateur, en utilisant une analyse en composantes indépendantes (ICA), un second sous-ensemble de sorties PCG filtrées de la pluralité de sorties PCG filtrées pour former le nombre M de transformations ICA filtrées ;
- transformer, par ledit au moins un processeur d'ordinateur, en utilisant l'analyse en composantes indépendantes (ICA), une première partie du second sous-ensemble de sorties PCG filtrées débruitées pour former le nombre M de transformations ICA filtrées débruitées ;
- compiler, par ledit au moins un processeur d'ordinateur, un nombre S d'une pluralité d'entrées de battements cardiaques de détection (DH), comprenant :

    i) le nombre M de sorties PCG filtrées,
    ii) le nombre M de sorties PCG filtrées débruitées,
    iii) le nombre M de transformations ICA filtrées et
    iv) le nombre M de transformations ICA filtrées débruitées ;

- détecter, par ledit au moins un processeur d'ordinateur, des emplacements de battement des battements dans chacune des entrées DH;
- calculer, par ledit au moins un processeur d'ordinateur, un score de confiance qui décrit une probabilité que les battements dans chaque entrée DH de la pluralité d'entrées DH représentent des battements cardiaques réels et non un bruit ;
- diviser, par ledit au moins un processeur d'ordinateur, la pluralité d'entrées DH en au moins deux groupes :

    i) un premier groupe d'entrées DH contenant des battements cardiaques fœtaux,
    ii) un deuxième groupe d'entrées DH contenant des battements cardiaques maternels ;

- sélectionner, par ledit au moins un processeur d'ordinateur, dans le premier groupe d'entrées DH, au moins une entrée DH fœtale particulière qui contient le battement cardiaque fœtal sur la base d'un premier score de confiance de ladite au moins une entrée DH fœtale particulière ; et
- sélectionner, par ledit au moins un processeur d'ordinateur, dans le second groupe d'entrées DH, au moins une entrée DH maternelle particulière qui contient le battement cardiaque maternel sur la base d'un second score de confiance de ladite au moins une entrée DH maternelle particulière.

2. Procédé mis en œuvre par ordinateur selon la revendication 1,
dans lequel K est égal à un nombre de la pluralité de capteurs acoustiques et L est égal à 6.

3. Procédé mis en œuvre par ordinateur selon la revendication 1 ou 2,
dans lequel chaque filtre de la pluralité de filtres passe-bande a une largeur de bande de 10 à 100 Hz et une gamme de fréquences de 5 à 110 Hz.

4. Procédé mis en œuvre par ordinateur selon l'une des revendications 1 à 3,
dans lequel la détection des emplacements de battement des battements dans chacune des entrées DH comprend les étapes consistant à :

- calculer, par ledit au moins un processeur d'ordinateur, un filtre de transformation prédéterminé ;
- répéter itérativement, par ledit au moins un processeur d'ordinateur, sur la base d'une longueur de fenêtre moyenne prédéterminée du filtre de transformation prédéterminé, les étapes consistant à :

    - identifier, dans chaque entrée DH, un sous-ensemble de pics ayant une forme prédéterminée et une taille prédéterminée et
    - sélectionner un groupe de pics dans le sous-ensemble de pics, le groupe de pics ayant la plus petite mesure de variance ;

- calculer, par ledit au moins un processeur d'ordinateur, des emplacements initiaux des battements.

5. Procédé mis en œuvre par ordinateur selon l'une des revendications 1 à 4, dans lequel le calcul du score de confiance comprend l'étape consistant à :

- générer, par ledit au moins un processeur d'ordinateur, un cardiogramme battement par battement pour chaque entrée DH sur la base des emplacements de battement.

6. Procédé mis en œuvre par ordinateur selon l'une des revendications 1 à 5, dans lequel la division de la pluralité d'entrées DH en le premier groupe d'entrées DH contenant des battements cardiaques fœtaux et le second groupe d'entrées DH contenant des battements cardiaques maternels, comprend les étapes consistant à:

- regrouper, par ledit au moins un processeur d'ordinateur, les battements de chaque entrée DH en fonction de chaque valeur de battement ; et
- affecter, par ledit au moins un processeur d'ordinateur, une entrée DH particulière ayant un taux de battements moyen plus élevé au premier groupe d'entrées DH contenant les battements cardiaques fœtaux.

7. Système informatique programmé spécifiquement, comprenant :

- au moins un ordinateur spécialisé, comprenant :

- une mémoire non transitoire, stockant électroniquement un code de programme exécutable par ordinateur particulier ; et
- au moins un processeur d'ordinateur qui, lorsqu'il exécute le code de programme particulier, devient un processeur d'ordinateur programmé spécifiquement qui est configuré pour effectuer au moins les opérations suivantes:

- recevoir une pluralité d'entrées de données de signaux de phonocardiogramme (PCG) provenant d'une pluralité de capteurs acoustiques ;
- filtrer numériquement, en utilisant une pluralité de filtres passe-bande, la pluralité de signaux d'entrée de données de signaux PCG pour former une pluralité de sorties PCG filtrées, la pluralité de filtres passe-bande comprenant un nombre L de filtres passe-bande, chaque filtre passe-bande délivrant un nombre K de sorties PCG filtrées ;

- débruiter par ondelettes un premier sous-ensemble de sorties PCG filtrées de la pluralité de sorties PCG filtrées pour former un nombre M de sorties PCG filtrées débruitées, M étant égal à L multiplié par K, le débruitage par ondelettes consistant à :

- déconstruire chaque sortie PCG filtrée pour générer une pluralité de coefficients de transformation, en faisant passer non relativement chaque sortie PCG filtrée par une succession de filtres passe-bas et passe-haut,
- identifier un sous-ensemble de coefficients de transformation porteurs de battements cardiaques parmi la pluralité de coefficients de transformation, et
- reconstruire le sous-ensemble de coefficients de transformation porteurs de battements cardiaques pour former le nombre M de sorties PCG filtrées débruitées ;
- transformer, en utilisant une analyse en composantes indépendantes (ICA), un second sous-ensemble de sorties PCG filtrées de la pluralité de sorties PCG filtrées pour former le nombre M de transformations ICA filtrées ;
- transformer, en utilisant l'analyse en composantes indépendantes (ICA), une première partie du second sous-ensemble de sorties PCG filtrées débruitées pour former le nombre M de transformations ICA filtrées débruitées ;
- compiler un nombre S d'une pluralité d'entrées de battements cardiaques de détection (DH), comprenant :

i) le nombre M de sorties PCG filtrées,
ii) le nombre M de sorties PCG filtrées débruitées,
iii) le nombre M de transformations ICA filtrées et
iv) le nombre M de transformations ICA filtrées débruitées;

- détecter des emplacements de battement des battements dans chacune des entrées DH ;
- calculer un score de confiance qui décrit une probabilité que les battements dans chaque entrée DH de la pluralité d'entrées DH représentent des battements cardiaques réels et non un bruit ;
- diviser la pluralité d'entrées DH en au moins deux groupes :

i) un premier groupe d'entrées DH contenant des battements cardiaques fœtaux,
ii) un deuxième groupe d'entrées DH contenant des battements cardiaques maternels ;

- sélectionner, dans le premier groupe d'entrées DH, au moins une entrée DH fœtale particulière qui contient le battement cardiaque fœtal sur la base d'un premier score de confiance de ladite au moins une entrée DH fœtale particulière ; et
- sélectionner, dans le second groupe d'entrées DH, au moins une entrée DH maternelle particulière qui contient le battement cardiaque maternel sur la base d'un second score de confiance de ladite au moins une entrée DH maternelle particulière.

8. Système informatique programmé spécifiquement selon la revendication 7,
dans lequel K est égal à un nombre de la pluralité de capteurs acoustiques et L est égal à 6.

9. Système informatique programmé spécifiquement selon la revendication 7 ou 8,
dans lequel chaque filtre de la pluralité de filtres passe-bande a une largeur de bande de 10 à 100 Hz et une gamme de fréquences de 5 à 110 Hz.

10. Système informatique programmé spécifiquement selon l'une des revendications 7 à 9,
dans lequel la détection des emplacements de battement des battements dans chacune des entrées DH comprend les étapes consistant à :

- calculer un filtre de transformation prédéterminé ;
- répéter itérativement, sur la base d'une longueur de fenêtre moyenne prédéterminée du filtre de transformation prédéterminé, les étapes consistant à:

- identifier, dans chaque entrée DH, un sous-ensemble de pics ayant une forme prédéterminée et une taille prédéterminée et
- sélectionner un groupe de pics dans le sous-ensemble de pics, le groupe de pics ayant la plus petite mesure de variance ;
- calculer des emplacements initiaux des battements.

11. Système informatique programmé spécifiquement selon l'une des revendications 7 à 10,
dans lequel le calcul du score de confiance comprend l'étape consistant à :

- générer un cardiogramme battement par battement pour chaque entrée DH sur la base des emplacements de battement.

12. Système informatique programmé spécifiquement selon l'une des revendications 7 à 11,
dans lequel la division de la pluralité d'entrées DH en le premier groupe d'entrées DH contenant des battements cardiaques fœtaux et le second groupe d'entrées DH contenant des battements cardiaques maternels, comprend les étapes consistant à :

- regrouper les battements de chaque entrée DH en fonction de chaque valeur de battement ; et
- affecter une entrée DH particulière ayant un taux de battements moyen plus élevé au premier groupe d'entrées DH contenant les battements cardiaques fœtaux.

*Fig. 1*

*Fig. 2*

*Fig. 3A*

*Fig. 3B*

*Fig. 4*

*Fig. 5*

Fig. 6

Fig. 7

EP 3 270 775 B1

Fig. 8

EP 3 270 775 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140005988 A1

- US 20120071744 A1

**Non-patent literature cited in the description**

- **HYVÄRINEN et al.** Independent component analysis: Algorithms and applications. *Neural Networks,* 2000, vol. 13 (4-5), 411-430